# EUROPÄISCHE PATENTANMELDUNG

(11) **EP 3 636 298 A1**
(43) Veröffentlichungstag der Anmeldung: **15.04.2020**
(21) Anmeldenummer: 18199475.7
(22) Anmeldetag: 10.10.2018
(51) Int. Cl.: A61M 5/142

(54) **EINSTECHVORRICHTUNG FÜR EINE VERABREICHUNGSVORRICHTUNG**

(71) Anmelder: Tecpharma Licensing AG, 3401 Burgdorf (CH)
(72) Erfinder: Streit, Ursina, 3322 Schönbühl (CH); Hostettler, Patrick, 3415 Hasle (CH); Hofer, Christophe, 3400 Burgdorf (CH); Margot, Roland, 3079 Worb (CH)

(57) **Zusammenfassung**

Die Erfindung betrifft eine Einstechvorrichtung (10) für ein Verabreichungsvorrichtung (1) umfassend ein elastisches Element (80), mit dem eine Vorspannkraft zum Einstechen erzeugbar ist; eine bewegliche Kanüleneinheit, welche mit der Vorspannkraft in eine Einstechrichtung vorgespannt ist oder in Einstechrichtung bewegbar ist; ein Sicherungselement (50), welches die vorgespannte Kanüleneinheit in einer Sicherungsstellung halten kann und einen thermosensitiven Bereich aufweist, welcher sich bei Erwärmung verformt, und ein Heizelement (41), mit dem der thermosensitive Bereich des Sicherungselements (50) erwärmbar ist, so dass sich das Sicherungselement (50) durch die Vorspannkraft verformt und dadurch die Kanüleneinheit aus der Sicherungsstellung wegbewegt werden kann. Weiter betrifft die Erfindung eine Verabreichungsvorrichtung (1) mit einem Mehrwegteil und einem Einwegteil, welches eine solche Einstechvorrichtung (10) umfasst.

## Beschreibung

### TECHNISCHES GEBIET

Die vorliegende Erfindung betrifft das Gebiet der medizinischen Verabreichungsvorrichtungen zur Verabreichung von flüssigen Substanzen, insbesondere von Medikamenten oder medizinischen Substanzen wie Insulin- und Hormonpräparationen mittels einer Kanüle, die in einen Körper eingebracht wird. Insbesondere bezieht sich die Erfindung auf eine Einstechvorrichtung, mit der eine Kanüle automatisch in einen Körper eingestochen werden kann, indem diese zum Beispiel aus einer Verabreichungsvorrichtung oder aus einem Kanülengehäuse ausgefahren wird. Nach dem Eindringen der Kanüle in einen Körper kann eine medizinische Substanz durch die Kanüle dosiert an den Körper abgegeben werden.

### HINTERGRUND DER ERFINDUNG

Zur Verabreichung einer medizinischen Substanz oder Flüssigkeit wird häufig eine Spritze, ein Injektionspen oder eine Infusionspumpe verwendet. Die Infusionspumpe kann an ein Infusionsset angeschlossen werden, um die Substanz oder Flüssigkeit dosiert in das Körpergewebe des Patienten abzugeben. Eine Möglichkeit der kontrollierten Substanzverabreichung ist die Abgabe durch eine tragbare Pumpe, welche der Benutzer auf sich trägt oder welche direkt auf seine Haut aufgeklebt wird. Letztere wird auch als Patch-Pumpe bezeichnet. In beiden Fällen wird die abzugebende Substanz von der Pumpe durch eine in den Körper des Benutzers eingebrachte Kanüle abgegeben. Das Einbringen der Kanüle kann manuell, zum Beispiel mittels eines bekannten Infusionssets, erfolgen. Alternativ zum manuellen Setzen einer Kanüle sind auch automatische Einstechvorrichtungen bekannt.

EP 1 549 382 B1 offenbart beispielsweise eine Einstechvorrichtung für eine Patch-Pumpe. Die Einstechvorrichtung umfasst eine flexible und eine steife Kanüle, wobei sich die steife Kanüle im Innern der flexiblen Kanüle befindet. Weiter umfasst die Einstechvorrichtung ein verschiebbares Mitnehmerelement, welches mit der flexiblen Kanüle verbunden ist und ein verschiebbares Rückzugselement, welches mit der steifen Kanüle verbunden ist. Wenn mittels elektrischer Betätigung ein "Shape Memory"-Element bewegt wird, wird ein Verriegelungselement verschoben, wodurch sich eine vorgespannte Einstechfeder entspannen kann und dadurch das Mitnehmerelement zusammen mit dem Rückzugselement aus dem Gehäuse hinausbewegt, wodurch die beiden Kanülen in die Haut des Patienten eingestochen werden. Das Mitnehmerelement umfasst radial von den Kanülen abragende flexible Finger, welche das Rückhalteelement beim Einstechvorgang mit dem Mitnehmerelement koppeln. Sobald jedoch das Mitnehmerelement vollständig ausgefahren ist, wird ein Formschluss aufgehoben und die flexiblen Finger können sich vom Rückhalteelement lösen. Dadurch kann sich eine koaxial zur Einstechfeder angeordnete und vorgespannte Rückzugsfeder entspannen und damit das Rückzugselement zusammen mit der steifen Kanüle zurück in das Gehäuse zum Halteelement hin verschieben. Somit wird die steife Kanüle aus der Haut herausgezogen, während die flexible Kanüle in der Haut verbleibt und einen Fluidpfad von einem Reservoir der Pumpe in die Haut ermöglicht.

Für die Freigabe oder das Auslösen des Einstechvorgangs werden "Shape Memory"-Elemente, mechanische Aktuatoren oder auch thermische Elemente verwendet wie beispielsweise in der US 9,220,838 B2 beschrieben. Diese offenbart eine Einstechvorrichtung, bei der eine Kanüle in einem Gehäuse durch eine Einstechfeder vorgespannt ist, wobei die Einstechfeder durch ein Rückhalteteil an der Entspannung und dem Vorschub der Kanüle gehindert wird. Das Rückhalteteil ist ein drehbarer Hebel, der seinerseits von einer weiteren Auslösefeder in Freigaberichtung mit einer Kraft beaufschlagt wird. Das drehbare Rückhalteteil wird durch einen Aktuator gegen die Kraft der Auslösefeder in Blockierstellung gehalten, wobei nach Betätigung des Aktuators und Freigabe des Rückhalteteils das Rückhalteteil durch die Auslösefeder aus der Blockierstellung in eine Freigabeposition gezogen wird, sodass die Einstechfeder sich entspannen kann und die Kanüleninsertion bewirken kann. In einer Ausführungsform kann der Aktuator zum Rückhalten des Rückhalteteils gegen die Kraft der Auslösefeder ein Hakenelement sein, welches mit einem Schmelzteil verbunden ist, das von einem Draht in Form einer Heizwendel umwickelt ist. Fliesst ein elektrischer Strom durch den Draht, schmilzt der Schmelzteil, sodass das Rückhalteteil bedingt durch die Kraft der Auslösefeder um eine Drehachse gedreht wird und die Einstechfeder funktional freigibt.

Solche bekannten Auslösemechanismen von Einstechvorrichtungen haben den Nachteil, dass sie einen teuren mechanischen Aktuator benötigen oder - im Falle eines thermischen Aktuators - eine aufwändige Konstruktion bedingen, welche viel Platz benötigt.

### DARSTELLUNG DER ERFINDUNG

Es ist eine Aufgabe der Erfindung, einfach und kostengünstig eine Auslösung einer Kanüleneinheit in einer Einstechvorrichtung zu ermöglichen. Diese Aufgabe wird gelöst durch eine Einstechvorrichtung und eine Verabreichungsvorrichtung gemäss den unabhängigen Ansprüchen. Bevorzugte Ausführungsformen sind Gegenstand der abhängigen Ansprüche.

Gemäss der Erfindung umfasst die Einstechvorrichtung ein elastisches Element, mit dem eine Vorspannkraft zum Einstechen erzeugbar ist, eine bewegliche Kanüleneinheit, welche mit der Vorspannkraft in eine Einstechrichtung vorgespannt ist oder in Einstechrichtung bewegbar ist, ein Sicherungselement, welches die vorgespannte Kanüleneinheit in einer Sicherungsstellung halten kann und einen thermosensitiven Bereich aufweist, welcher sich bei Erwärmung unter mechanischer Spannung verformt, und ein Heizelement, mit dem der thermosensitive Bereich des Sicherungselements erwärmbar ist. Dadurch kann sich der thermosensitive Bereich des Sicherungselements unter mechanischer, durch die Vorspannkraft hervorgerufener Spannung verformen. In Folge kann die Kanüleneinheit nicht mehr in der Sicherungsstellung gehalten werden und die Kanüleneinheit kann sich aus der Sicherungsstellung wegbewegen.

Erfindungsgemäss wird die Vorspannkraft eines elastischen Elements, welches die Kanüleneinheit zum Einstechen vorspannt, zumindest teilweise auf ein Sicherungselement übertragen. Diese zumindest teilweise Übertragung der Vorspannkraft erfolgt vorzugsweise indirekt über mindestens ein weiteres Element wie beispielsweise ein Übertragungselement oder Rückhalteelement. Jedoch kann genauso gut die Vorspannkraft direkt von der Kanüleneinheit auf das Sicherungselement übertragen werden. In jedem Fall ist das Sicherungselement dazu ausgebildet, die vorgespannte Kanüleneinheit entgegen der Vorspannkraft in einer Sicherungsstellung zu halten. Die Kanüleneinheit kann mittels Kraftschluss, Formschluss oder einer Kombination von Kraft- und Formschluss durch das Sicherungselement gehalten werden. Vorzugsweise besteht eine formschlüssige Verbindung zwischen Sicherungselement und Kanüleneinheit. Hierzu weist das Sicherungselement beispielsweise eine Lasche, einen Haken, einen Zapfen oder eine Aufnahme für ein entsprechend geformtes Gegenelement auf. Falls zwischen Sicherungselement und Kanüleneinheit ein weiteres Element wie ein Übertragungselement oder Rückhalteelement angeordnet ist, sind die oben genannten Verbindungen vorzugsweise sowohl zwischen Sicherungselement und dem weiteren Element wie auch zwischen dem weiteren Element und Kanüleneinheit realisiert. Durch die mindestens teilweise Übertragung der Vorspannkraft vom elastischen Element auf das Sicherungselement steht dieses unter einer mechanischen Spannung in der Form einer Zug-, Druck-, Biege-, Torsionsspannung oder einer Kombination dieser Spannungsarten (mehrachsige Spannungszustände). Vorzugsweise ist das Sicherungselement mit einem Ende an einer Basis, einem Support oder Halter befestigt und wirkt mit dem anderen Ende direkt oder indirekt über ein weiteres Element mit der Kanüleneinheit zusammen.

Um den Einstechvorgang auszulösen, so dass die Kanüle in die Haut des Benutzers eingestochen wird, wird ein thermosensitiver Bereich des Sicherungselements mittels eines Heizelements erwärmt. Infolge der Erwärmung verformt sich der thermosensitive Bereich oder reisst auseinander. Dadurch kann die Kanüleneinheit nicht länger durch das Sicherungselement in der Sicherungsstellung gehalten werden. Das bedeutet, die Sperr- oder Haltefunktion des Sicherungselements wird aufgehoben. Das vorgespannte elastische Element kann sich in der Folge mindestens teilweise entspannen und die Kanüleneinheit aus der Sicherungsstellung in Einstechrichtung bewegen. Dabei bewegt das elastische Element die Kanüleneinheit entweder direkt oder indirekt über ein Zwischenelement in Einstechrichtung. Die Kanüleneinheit bewegt sich vorzugsweise zusammen mit einer Kanüle in eine Einstechstellung, in der die Kanüle mit ihrem einstechseitigen Ende in die Haut des Benutzers eingestochen ist. Ein Fluid, welches beispielsweise an einem dem Einstichort abgewandten Ende der Kanüle in diese eintritt, kann durch die Kanüle hindurchfliessen und tritt an der Austrittsöffnung der Kanüle unter der Haut des Benutzers aus.

Somit benötigt die Auslösung des Einstechvorgangs nur einen Aktor in Form eines Heizelements. Das bedeutet, die durch die Vorspannung des elastischen Elements gespeicherte Energie wird nicht nur zum Einstechen der Kanülen sondern auch für den Auslösevorgang verwendet. Dadurch ist für die Freigabe der Kanüleneinheit aus der Sicherungsstellung nicht eigens ein Aktor wie beispielsweise ein Stellmotor, eine Solenoidspule oder eine zusätzliche mechanische Feder nötig, welche das Sicherungselement bewegt. Es wird mit der erfindungsgemässen Einstechvorrichtung direkt ein Teil der zum Einstechen vorgesehenen Vorspannkraft oder Vorspannenergie verwendet, um das Sicherungselement zu bewegen und damit den Einstechvorgang auszulösen. Zudem lässt sich die Auslösung mit dem Heizelement mit weniger Bauteilen realisieren als beispielsweise eine Auslösung mit einer Solenoidspule oder einem sonstigen elektromechanischen Aktuator. Ausserdem ermöglicht die Auslösung mit dem Heizelement mit der Vorspannkraft eine sehr kompakte Bauweise, da das Heizelement sehr klein, nämlich im Bereich von wenigen Millimetern Baugrösse, ausgeführt werden kann. Dadurch ist eine einfache, kostengünstige und auch kompakte Konstruktion der Auslösemechanik und damit der Einstechvorrichtung ermöglicht.

Vorzugsweise weist ein Element der Kanüleneinheit eine schräg zur Einstechrichtung ausgerichtete Fläche auf und das Sicherungselement oder ein mit dem Sicherungselement zusammenwirkendes Übertragungselement weist eine schräg zur Einstechrichtung ausgerichtete Sicherungsfläche auf. Wenn sich das Sicherungselement vorzugsweise unter Spannung durch Erwärmung verformt oder reisst und dadurch die Kanüleneinheit freigibt, wird vorzugsweise die schräge Fläche der Kanüleneinheit auf die Sicherungsfläche gedrückt, wodurch die schräg zur Einstechrichtung und damit zur Bewegungsrichtung der Kanüleneinheit ausgerichteten Flächen aufeinander abrutschen oder aufeinander abgleiten, so dass die durch das elastische Elemente angetriebene Kanüleneinheit das Sicherungselement oder das Übertragungselement zur Seite bewegen kann.

Unter dem Begriff "Verabreichungsvorrichtung" wird in der vorliegenden Beschreibung eine Vorrichtung verstanden, bei der die Injektionsnadel permanent bzw. über einen längeren Zeitraum von mehreren Stunden im Patienten verbleibt. Eine solche Verabreichungsvorrichtung wird auch als Infusionssystem bezeichnet. Somit wird bei einem Infusionssystem im Unterschied zu einem Injektionssystem oder Injektor die Injektionsnadel nach Abgabe der medizinischen Substanz nicht unmittelbar aus dem Gewebe des Benutzers entfernt. Eine Ausnahme zu dieser Definition bildet sogenannte Patchinjektoren, welche im Zusammenhang mit der Erfindung ebenfalls unter den Begriff "Verabreichungsvorrichtung" fallen. Ein Patchinjektor ist auf die Haut des Benutzers aufklebbar, wobei eine Kanüle oder Injektionsnadel in die Haut des Benutzers eingestochen wird. Der Patchinjektor verbleibt auf der Haut, üblicherweise mehrere Minuten, bis die medizinische Substanz aus dem Patchinjektor an den Benutzer abgegeben worden ist.

Vorzugsweise handelt es sich bei der erfindungsgemässen Verabreichungsvorrichtung um ein Infusionssystem. Weiter wird der Einstechvorgang mit der erfindungsgemässen Einstechvorrichtung vorzugsweise elektronisch mittels eines Steuerbefehls ausgelöst.

Das elastische Element zum Erzeugen der Vorspannkraft kann beispielsweise als mechanische Zug-, Druck- oder Torsionsfeder aber auch in Form einer komprimierbaren Flüssigkeit oder eines elastischen Kunststoffs wie beispielsweise einem Elastomer ausgebildet sein.

Die Kanüleneinheit umfasst vorzugsweise mindestens eine Kanüle, welche unter die Haut des Benutzers eingebracht werden kann. Weiter umfasst die Kanüleneinheit bevorzugt einen Kanülenhalter, auf dem die Kanüle befestigt ist, und welche relativ zu einem Kanülengehäuse oder einer Basis der Verabreichungsvorrichtung zusammen mit einem einstechseitigen Bereich der Kanüle bewegbar ist. Durch die Bewegung des Kanülenhalters mit der Kanüle kann ein einstechseitiger Bereich der Kanüle in die Haut des Benutzers eingestochen werden.

In einer bevorzugten Ausführung umfasst die Kanüleneinheit eine Hart- und eine Softkanüle, welche koaxial zueinander angeordnet sind. In einer bevorzugten Ausführung wird die Hartkanüle zusammen mit der Softkanüle in die Haut eingestochen und anschliessend wird die Hartkanüle wieder aus der Einstechstelle heraus gezogen, während die Softkanüle in der Haut verbleibt. Dabei umgibt zumindest beim Einstechvorgang entweder die Softkanüle die Hartkanüle oder die Hartkanüle umgibt die Softkanüle. Vorzugsweise liegt die äussere Kanüle (Hart- oder Softkanüle) mit ihrem Innenumfang dichtend am Aussenumfang der Hartkanüle an, sodass kein Fluid hindurchtreten kann. Dabei ist die innere Kanüle (Hart- oder Softkanüle) vorzugsweise innerhalb der äusseren Kanüle verschiebbar. In einer bevorzugten Ausführung sind Soft- und Hartkanüle bevorzugt biegbar, also können z.B. in ihrer Längsrichtung gebogen oder gekrümmt werden, so dass eine Bewegungsrichtung der Kanüleneinheit von einer Bewegungsrichtung der Kanülenspitze verschieden sein kann. Falls ein Verlauf der Hart- und Softkanüle einen gekrümmten oder bogenförmigen Verlauf annimmt, wird die Hartkanüle beim Einstechvorgang bevorzugt plastisch deformiert. In einer alternativen Ausführung kann die Hartkanüle beim Einstechvorgang jedoch auch im elastischen Bereich verformt werden. Dabei können die Hart- und Softkanüle initial gerade ausgeformt sein und bei der Montage oder beim Einbau in die Kanülenhalter gebogen werden.

In einer alternativen Ausführung umfasst die Kanüleneinheit nur eine Softkanüle und eine Einstechnadel, welche koaxial in der Softkanüle angeordnet ist und die Softkanüle im Querschnitt nur teilweise ausfüllt, so dass immer noch ein Fluid neben der Einstechnadel durchfliessen kann. In diesem Fall weist die Softkanüle bevorzugt eine Verzweigung auf, wobei die Einstechnadel durch einen ersten Kanal der Softkanüle zurückgezogen wird und das Fluid durch einen zweiten Kanal der Softkanüle zum einstechseitigen Ende der Softkanüle fliessen kann. Unabhängig von der Anordnung ist die Softkanüle vorzugsweise aus einem flexiblen Material wie beispielsweise Kunststoff und die Hartkanüle vorzugsweise aus einem steifen Material wie beispielsweise Stahl gefertigt.

In der folgenden Beschreibung wird eine Seite oder Richtung der Einstechvorrichtung, welche gegen die Spitze der Kanüle oder dem einstechseitigen Ende der Kanüle weist, als "einstechseitig" oder proximal bzw. als "Einstechrichtung" bezeichnet. Eine Seite oder Richtung hingegen, welche gegen eine Abschlusswandung der Verabreichungsvorrichtung weist und die der Einstechstelle abgewandt ist, wird als distal bzw. als distale Richtung bezeichnet. Somit liegt ein einstechseitiger (proximaler) Bereich gegenüber einem distalen Bereich und eine Einstechrichtung (proximale Richtung) weist entgegen einer distalen Richtung.

Die Einstechrichtung entspricht der Richtung in der die Kanüleneinheit beim Einstechvorgang bewegt wird. Die Einstechrichtung kann von der Richtung, in der die Kanülenspitze für das Einstechen in die Haut des Benutzers bewegt wird, abweichen. So kann beispielsweise ein einstechseitiger Bereich der Kanüle zwischen Kanülenhalter und einstechseitigem Ende der Kanüle umgelenkt werden, so dass die Kanüle im einstechseitigen Bereich einen gekrümmten oder bogenförmigen Verlauf annimmt. In einem solchen Fall ist die Einstechrichtung der Kanüleneinheit unterschiedlich zur Bewegungsrichtung des einstechseitigen Bereichs der Kanüle.

Die Kanüleneinheit ist vorzugsweise mit der Vorspannkraft in die Einstechrichtung vorgespannt. Genauso gut kann jedoch ein Antriebselement mit der Vorspannkraft vorgespannt sein, welches bei seiner Auslösung auf die Kanüleneinheit wirkt und diese in Einstechrichtung bewegt. Dieses Antriebselement kann separat und getrennt von der Kanüleneinheit ausgebildet sein. In beiden Fällen ist die Kanüleneinheit durch die Vorspannkraft des elastischen Elements bewegbar.

Das Sicherungselement kann die vorgespannte Kanüleneinheit in der Sicherungsstellung unbeweglich halten, so dass die Kanüleneinheit nicht durch die Vorspannkraft bewegt werden kann. Vorzugsweise wird die Vorspannkraft indirekt, bevorzugt über ein weiteres Element wie ein Übertragungselement oder Rückhalteelement oder über mehrere solche Elemente vom elastischen Element auf das Sicherungselement übertragen. Dadurch hält das Sicherungselement über ein oder mehrere Element die Kanüleneinheit in der Sicherungsstellung.

Das Sicherungselement verformt sich bei Erwärmung oder reisst ganz auseinander. Das Material des Sicherungselements muss dabei nicht zwingend flüssig werden respektive seinen Schmelzpunkt erreichen. So geht zum Beispiel beim Überschreiten der Glasübergangstemperatur ein fester amorpher Stoff oder festes Glas oder Polymer in einen gummiartigen bis zähflüssigen Zustand über. Bereits ein Aufweichen des Materials kann genügen, damit sich das Sicherungselement durch die Vorspannkraft verformt und dadurch die Kanüleneinheit aus der Sicherungsstellung wegbewegbar ist bzw. die Haltefunktion des Sicherungselements aufgehoben ist. Vorzugsweise wird das Sicherungselement ab einer Temperatur von 80°C aufgeweicht, geschwächt, verformt oder auseinandergerissen, so dass die Kanüleneinheit aus der Sicherungsstellung wegbewegbar ist.

In einer alternativen Ausführung besteht aber auch die Möglichkeit, dass sich Sicherungselement bei Erwärmung zusammenzieht, so dass die Kanüleneinheit aus der Sicherungsstellung wegbewegt werden kann bzw. die Haltefunktion des Sicherungselements aufgehoben ist. In diesem Fall ist das Sicherungselement vorzugsweise aus einem Formgedächtnispolymer (shape-memory polymer) gebildet, welches sich bei Erwärmung zusammenzieht oder verkürzt.

Der Begriff "Medikament" oder "medizinische Substanz" umfasst in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

In einer bevorzugten Ausführung weist die Einstechvorrichtung ein bewegbares Rückhalteelement auf, welches funktional zwischen der Kanüleneinheit oder dem elastischen Element und dem Sicherungselement angeordnet ist und welches die Vorspannkraft vom elastischen Element auf das Sicherungselement übertragen kann. Dabei bedeutet "funktional", dass sich das Rückhalteelement in einer kinematischen Kette zwischen elastischem Element und Sicherungselement befindet. Das Rückhalteelement muss also nicht zwingend örtlich oder in Bezug auf die strukturelle Anordnung zwischen dem elastischen Element und dem Sicherungselement angeordnet sein. Unabhängig davon, wie das Rückhalteelement angeordnet ist überträgt dieses mindestens teilweise die Vorspannkraft vom elastischen Element auf das Sicherungselement, wobei das elastische Element bevorzugt die Vorspannkraft zuerst auf die Kanüleneinheit überträgt.

Bevorzugt ist das Rückhalteelement derart ausgebildet, dass es in der Sicherungsstellung die Kanüleneinheit blockieren kann bzw. an einer Bewegung hindern kann, indem das Rückhalteelement wiederum durch das Sicherungselement blockiert bzw. an einer Bewegung gehindert wird. Das Rückhalteelement wirkt also vorzugsweise direkt mit der Kanüleneinheit zusammen und fungiert als Verbindungsglied zwischen der Kanüleneinheit und Sicherungselement. Das bietet den Vorteil, dass mittels des Rückhalteelements die Übertragung der Vorspannkraft auf das Sicherungselement definiert und eingestellt werden kann. Mit dem Rückhalteelement kann also eine mechanische Übersetzung oder Untersetzung realisiert werden. In Abhängigkeit der geometrischen Dimensionen (Hebelarmlängen) und dem Ort der Lagerung in Bezug auf den Ort der Kraftaufnahme kann die Vorspannkraft übersetzt oder untersetzt werden. Somit kann die Vorspannkraft, welche auf das Sicherungselement wirkt, mit dem Rückhalteelement eingestellt werden, was eine platzsparende Dimensionierung des Sicherungselements ermöglicht.

Das Rückhalteelement kann über eine kraftschlüssige oder formschlüssige Verbindung oder mit einer Kombination einer kraft- und formschlüssigen Verbindung mit der Kanüleneinheit und dem Sicherungselement zusammenwirken. Vorzugsweise weist das Rückhalteelement in einem Winkel eine schräg zur Einstechrichtung angeordnete Sicherungsfläche auf, welche in der Sicherungsstellung mit der Kanüleneinheit zusammenwirkt und dadurch die Kanüleneinheit in Position hält. Der Winkel, in dem die Sicherungsfläche zur Einstechrichtung ausgerichtet ist, beeinflusst die Höhe der Kraft, welche von der vorgespannten Kanüleneinheit auf das Sicherungselement übertragen wird. Somit ist durch die Wahl des Winkels diese Kraft einstellbar. Weiter ist das Rückhalteelement in einer bevorzugten Ausführung mittels einer formschlüssigen Verbindung mit dem Sicherungselement gekoppelt.

Alternativ besteht auch die Möglichkeit, dass die Einstechvorrichtung kein Rückhalteelement aufweist und die Vorspannkraft direkt vom elastischen Element bzw. von der Kanüleneinheit auf das Sicherungselement übertragen wird. In diesem Fall wirkt die Kanüleneinheit direkt ohne zusätzliches Element oder Bauteil mit dem Sicherungselement zusammen.

Bevorzugt ist das Rückhalteelement als rotierbarer Hebel ausgebildet, welcher um eine Drehachse schwenkbar ist, um die Kanüleneinheit aus der Sicherungsstellung freizugeben. Dabei ist das Rückhalteelement vorzugsweise an einer Basis, einem Support oder Halter drehbar gelagert. Mit einem Rückhalteelement als drehbarer Hebel kann der Auslösemechanismus kompakt konstruiert werden. Der Hebel weist bevorzugt an einem ersten Ende eine schräg zur Einstechrichtung ausgerichtete Sicherungsfläche auf und an einem zweiten Ende ein Kopplungselement, welches mit dem Sicherungselement koppelbar ist. Vorzugsweise wirkt die Sicherungsfläche in der Sicherungsstellung mit einer schrägen Fläche der Kanüleneinheit zusammen, so dass die Kanüleneinheit durch das Rückhalteelement mittels Formschluss in Position gehalten wird und sich nicht bewegen kann.

Alternativ besteht auch die Möglichkeit, dass das Rückhalteelement nur verschiebbar ausgeführt ist oder sowohl verschiebbar wie auch drehbar ist.

Mit Vorteil ist das Rückhalteelement mindestens durch einen Teil der vom elastischen Element erzeugten Vorspannkraft zum Einstechen bewegbar. In einer bevorzugten Ausführung ist das Rückhalteelement ausschliesslich durch die Vorspannkraft bewegbar. Das bedeutet, wenn durch Erwärmung des Sicherungselements die Haltefunktion des Sicherungselements aufgehoben wird, kann das Rückhalteelement vorzugsweise allein durch die Vorspannkraft bewegt werden. Es ist somit kein zusätzlicher Aktuator nötig, um das Rückhalteelement zu bewegen. Das ermöglicht eine einfache und kostengünstige Konstruktion.

Vorzugsweise wird bei einer Auslösung das Rückhalteelement über seine Sicherungsfläche von einer schrägen Fläche der Kanüleneinheit weggedrückt, wodurch das Rückhalteelement aus dem Bewegungsraum der Kanüleneinheit wegbewegt wird. Die Kanüleneinheit kann sich dann vorzugsweise in Einstechrichtung wegbewegen, um den Einstechvorgang auszuführen.

Bevorzugt weist das Sicherungselement eine Schrägfläche auf, welche in der Sicherungsstellung der Kanüleneinheit durch die Vorspannkraft derart mit einer Gegenfläche zusammenwirkt, dass mindestens ein Teil des thermosensitiven Bereichs des Sicherungselements an das Heizelement gedrückt wird. Dabei befindet sich die Gegenfläche vorzugsweise auf einer Basis, einem Support oder einem Halter des Heizelements oder des Sicherungselements. Vorzugsweise ist das Sicherungselement beweglich relativ zu einem Halter des Sicherungselements gelagert. Das bedeutet, das Sicherungselement kann sich zumindest im Bereich von einigen Millimetern, vorzugsweise einige zehntel Millimeter, relativ zum Halter des Sicherungselements bewegen.

Vorzugsweise wird das Sicherungselement aufgrund der Vorspannkraft vom elastischen Element mit seiner Schrägfläche gegen die Gegenfläche gedrückt. Dadurch rutsch oder gleitet das Sicherungselement mit seiner Schrägfläche an der Gegenfläche ab, wodurch sich das Sicherungselement minimal, vorzugsweise weniger als ein Millimeter, besonders bevorzugt weniger als ein Zehntel Millimeter, in Richtung Heizelement bewegt, so dass mindestens ein Teil des thermosensitiven Bereichs gegen das Heizelement gedrückt wird. Dadurch wird vermieden, dass zwischen thermosensitiven Bereich und dem Heizelement ein Luftspalt oder Zwischenraum verbleibt, welcher isolierend wirkt und ein Erwärmen des thermosensitiven Bereichs behindern kann. Die Schrägfläche am Sicherungselement und die Gegenfläche ermöglichen somit ein effizientes Erwärmen des thermosensitiven Bereichs durch Wärmeübergang. Die Erwärmung des thermosensitiven Bereichs erfolgt bevorzugt also nicht mittels Konvektion.

Vorzugsweise ist die Schrägfläche am Sicherungselement als Rampe ausgeführt, welche gegenüber einer Längsachse des Sicherungselements einen Winkel zwischen 60° und 80°, vorzugsweise 70°, aufweist. Bevorzugt ist die Gegenfläche auf dem Halter als Rampe mit dem gleichen Winkel ausgebildet. Weiter befindet sich in einer bevorzugten Ausführung auf beiden Längsseiten des Sicherungselements eine Schrägfläche, wodurch sich das Sicherungselement ohne sich zu drehen oder zu verkannten relativ zum Halter bewegt werden kann.

Vorzugsweise ist das Sicherungselement gegenüber dem Heizelement als separates Bauteil ausgebildet. Das Sicherungselement umfasst also keine eingebaute oder integrierte Heizung, welche als Heizelement dient. Hingegen ist das Heizelement als separates vom Sicherungselement getrenntes Bauteil ausgebildet oder ist in einem separaten Bauteil integriert. Dadurch kann das Heizelement rasch montiert und ausgewechselt werden. Dabei ist das Sicherungselement vorzugsweise aus einem amorphen Kunststoff gebildet wie beispielsweise Polystyrol, Polyvinylchlorid oder Polycarbonat. Vorzugsweise ist das Sicherungselement hauptsächlich aus ABS, einem PC-ABS-Polymerblend oder einem glasfaserverstärktem ABS gebildet. Aufgrund der thermischen Eigenschaften von amorphen Kunststoffen kann das Sicherungselement besonders effizient Erwärmt werden.

In einer alternativen Ausführung besteht auch die Möglichkeit, dass das Heizelement in das Sicherungselement integriert ist. Ein Nachteil dieser Ausführung ist, dass das integrierte Heizelement beschädigt werden kann, wenn sich das Sicherungselement durch die Wärme des Heizelements zu verformen beginnt. Es besteht dann die Gefahr, dass das beschädigte Heizelement das Sicherungselement nicht mehr genügend weiter erwärmen kann, so dass sich das Sicherungselement nicht ausreichend verformen kann und in Folge die Auslösung der Kanüleneinheit nicht korrekt funktioniert.

In einer bevorzugten Ausführung ist das Heizelement in eine Leiterplatte integriert und bevorzugt ist das Heizelement als elektrischer Widerstand realisiert. Wird dieser bestromt, erwärmt sich der Widerstand und kann den thermosensitiven Bereich des Sicherungselements erwärmen. Das Heizelement ist vorzugsweise flächenförmig ausgebildet. Bevorzugt umfasst das Heizelement eine Fläche zwischen 1 mm² und 2.5 mm² und eine Dicke zwischen 10 und 50 µm.

In einer bevorzugten Ausführung wird das Heizelement aus einer Polymerpaste (Carbon Conductive Composition) gebildet, welche zusammen mit der Leiterbahngeometrie der Leiterplatte einen spezifischen elektrischen Widerstand ergibt. Der Widerstand beträgt dabei vorzugsweise zwischen 10 und 40 Ohm und die erzielte Wärmeleistung liegt vorzugsweise zwischen 200 und 700 mW.

Alternativ besteht auch die Möglichkeit, dass das Heizelement beispielsweise als Heizdraht oder Heizwendel ausgeführt ist.

Bevorzugt weist das Sicherungselement eine längliche Form mit einer Verjüngung im thermosensitiven Bereich auf und ist derart angeordnet ist, dass sich die Verjüngung und das Heizelement zumindest teilweise überlappen. Somit kann das Sicherungselement durch das Heizelement im Bereich der Verjüngung erwärmt oder geschmolzen werden. Vorzugsweise weist das Sicherungselement im Bereich der Verjüngung gegenüber dem übrigen Bereich eine kleinere Querschnittsfläche auf. Dadurch kann der thermosensitive Bereich rasch und effizient erwärmt oder geschmolzen werden. Vorzugsweise liegt das Sicherungselement mit einer Fläche im Bereich der Verjüngung auf einer Fläche des Heizelements auf, so dass die Wärmeübertragung vom Heizelement auf das Sicherungselement besonders effizient erfolgen kann.

In einer bevorzugten Ausführung umfasst die Kanüleneinheit eine erste und eine zweite Kanüle, wobei die zweite Kanüle durch eine zweite Vorspannkraft von einem zweiten elastischen Element entgegen der Einstechrichtung vorspannbar ist. Dabei ist bevorzugt die erste Kanüle aus einem flexiblen Material wie beispielsweise einem flexiblen Kunststoff gefertigt und die zweite Kanüle aus einem gegenüber dem flexiblen Material steiferen Material wie beispielsweise Stahl gefertigt. Der flexible Kunststoff der ersten Kanüle kann beispielsweise ein Fluorpolymer wie FEP sein. Vorzugsweise wird die erste Kanüle als Softkanüle und die zweite Kanüle als Hardkanüle bezeichnet. In einer bevorzugten Ausführung ist das steifere Material jedoch noch biegbar, so dass die erste wie auch die zweite Kanüle umgelenkt werden können. Das erste elastische Element dient vorzugsweise dazu die Kanüleneinheit inklusive der ersten und zweiten Kanüle für eine Einstechbewegung mit der Kanüle zu bewegen, während das zweite elastische Element vorzugsweise dazu dient, die zweite Kanüle nach einem Einstechen in die Haut aus der Haut zurückzuziehen.

Vorzugsweise ist die steifere Kanüle koaxial zur flexiblen Kanüle angeordnet und befindet sich innerhalb der flexiblen Kanüle. In einer alternativen Ausführung kann jedoch die flexible Kanüle innerhalb der steiferen Kanüle platziert sein.

In einer bevorzugten Ausführung werden die steifere und flexible Kanüle mit der Vorspannkraft vom ersten elastischen Element zusammen in die Haut eingestochen und anschliessend wird mit der Vorspannkraft des zweiten elastischen Elements die steifere Kanüle wieder zurückgezogen, während die flexible Kanüle in der Haut verbleibt. Die flexible Kanüle wird dabei von einer verbleibenden Kraft des ersten elastischen Elementes in Position gehalten. Alternativ kann die Kanülenhalterung der flexiblen Kanüle mit mittels einer Verankerung zum Support oder Basis in Position gehalten werden. Ein Fluid kann dann beispielsweise aus einem Reservoir durch ein distales Ende der steiferen Kanüle in diese eintreten und durch die steifere Kanüle in die flexible Kanüle und von dieser in den Körper des Benutzers fliessen.

Alternativ besteht aber auch die Möglichkeit, dass beim Einstechvorgang nur eine einzelne Kanüle in die Haut des Benutzers eingeführt wird. Diese ist mit Vorteil zumindest teilweise aus einem steifen Material, um eine rasche Insertion zu ermöglichen.

Vorzugsweise ist die Kanüleneinheit mit der ersten und der zweiten Kanüle und dem zweiten elastischen Element von der Sicherungsstellung in eine Einstechstellung bewegbar und die zweite Kanüle ist durch die zweite Vorspannkraft wieder aus der Einstechstellung wegbewegbar, wobei die erste Kanüle in der Einstechstellung verbleibt. Dabei wird die Kanüleneinheit in Einstechrichtung von der Sicherungsstellung in die Einstechstellung bewegt, während mindestens ein Bereich der Kanüle nicht zwingend in Einstechstellung bewegt werden muss. Wie weiter oben ausgeführt, kann die Kanüle abgelenkt oder gebogen werden. Die Kanülenspitze bewegt sich in diesem Fall während dem Einstechvorgang vorzugsweise in eine andere Richtung als die übrigen Elemente der Kanüleneinheit.

Bevorzugt sind das erste elastische Element und das zweite elastische Element je als mechanische Feder ausgebildet. Mit einer mechanischen Feder vorzugsweise in Form einer Druck- oder Zugfeder ist durch deren Komprimierung bzw. Expansion auf einfache Art eine Vorspannkraft erzeugbar. In einer alternativen Ausführung können die elastischen Elemente auch in Form von Elastomerelementen realisiert sein.

In einer bevorzugten Ausführung sind die Federn koaxial zueinander angeordnet. So kann beispielsweise eine erste Feder, welche die gesamte Kanüleneinheit mit der ersten und der zweiten Kanüle in eine Einstechrichtung bewegen kann, innerhalb einer zweiten Feder angeordnet sein, welche die zweite Kanüle entgegen der Einstechrichtung bewegen kann. Jedoch können in einer alternativen Ausführung die erste und die zweite Feder auch vertauscht angeordnet sein. Das erlaubt eine kompakte Konstruktion der Einstechvorrichtung. Alternativ können die Federn auch hintereinander oder nebeneinander angeordnet sein.

Zudem ist eine der zwei Federn vorzugsweise in Längsrichtung der Feder mindestens teilweise innerhalb der anderen der zwei Federn angeordnet, bevorzugt konzentrisch. Die Federn sind also in Längsrichtung oder axialer Richtung nicht hintereinander sondern bevorzugt ineinander angeordnet. Dadurch lässt sich die Einstechvorrichtung in Bezug auf die Längsrichtung besonders kompakt konstruieren.

Bevorzugt umfasst die Einstechvorrichtung einen Positionssensor oder Positionsdetektor, mit dem bestimmbar ist, ob sich die Kanüleneinheit in der Einstechstellung befindet und welcher über einen Kanülenhalter der Kanüleneinheit betätigbar ist. Bevorzugt weist der Kanülenhalter eine Kontaktfläche in der Form einer Anschrägung auf, durch die ein bewegliches Element des Positionssensors in der Einstechstellung verstellt wird, wodurch ein Sensorsignal erzeugbar ist. Der Kanülenhalter dient somit als Auslöseelement für den Positionssensor.

Der Kanülenhalter ist vorzugsweise dazu ausgebildet die Kanüle unbeweglich zu halten. Er ist bevorzugt zusammen mit der Kanüle relativ zu einer Basis, einem Support oder Halter bewegbar, insbesondere verschiebbar.

Die Erfindung betrifft weiter eine Verabreichungsvorrichtung, insbesondere eine Infusionspumpe, umfassend einen Einwegteil und einen mit dem Einwegteil koppelbaren Mehrwegteil. Dabei umfasst das Mehrwegteil eine Antriebseinheit und das Einwegteil ein Reservoir zur Aufnahme einer medizinischen Substanz und eine Einstechvorrichtung wie oben beschrieben, welche in Fluidverbindung mit dem Reservoir steht. Dabei dient das Einwegteil zum einmaligen Gebrauch. Das bedeutet, dass das Einwegteil nach einer bestimmten Zeit oder wenn die gesamte medizinische Substanz verabreicht wurde vom Mehrwegteil gelöst und entsorgt wird. Das Mehrwegteil dient zur mehrmaligen Verwendung und kann mit einem neu eingesetzten Einwegteil erneut gekoppelt werden.

In einer bevorzugten Ausführung ist die Verabreichungsvorrichtung als Patch-Pumpe ausgebildet. Eine Patch-Pumpe ist eine Infusionspumpe, bei der das Infusionsset und die eigentliche Pumpe mit der gesamten Mechanik in einem gemeinsamen Gehäuse kombiniert sind. Die Patch-Pumpe wird direkt auf die Haut des Benutzers geklebt. Die Einstellungen und Steuerung der Patch-Pumpe erfolgt dabei über ein mobiles Steuergerät, insbesondere über einen sogenannten "Personal Diabetes Manager" (PDM), welches mit der Patch-Pumpe drahtlos kommuniziert. Dabei ist das Gehäuse der Patch-Pumpe vorzugsweise zweiteilig ausgeführt, mit einem oben beschriebenen Einwegteil und einem Mehrwegteil.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt eine perspektivische Gesamtansicht einer Patch-Pumpe, welche einen Einweg- und einem Mehrwegteil aufweist;
- Fig. 2: zeigt eine perspektivische Ansicht des Einwegteils mit der erfindungsgemässen Einstechvorrichtung;
- Fig. 3: zeigt eine erfindungsgemässe Einstechvorrichtung;
- Fig. 4: zeigt eine Explosionsdarstellung der Einstechvorrichtung aus Figur 3;
- Fig. 5: zeigt eine Seitenansicht der Einstechvorrichtung, in der sich die Einstecheinheit in einer Sicherungsstellung befindet;
- Fig. 6: zeigt eine Schnittansicht der Einstechvorrichtung in der Sicherungsstellung, wobei der Schnitt horizontal durch die Einstechvorrichtung verläuft;
- Fig. 7: zeigt eine Seitenansicht, in der sich die Einstecheinheit in einer Einstechstellung befindet;
- Fig. 8: zeigt eine Schnittansicht in der Einstechstellung, wobei der Schnitt horizontal durch die Einstechvorrichtung verläuft;
- Fig. 9: zeigt eine Seitenansicht, in der sich die Einstecheinheit in einer Rückzugsstellung befindet;
- Fig. 10: zeigt eine Schnittansicht in der Rückzugsstellung, wobei der Schnitt horizontal durch die Einstechvorrichtung verläuft;
- Fig. 11: zeigt eine perspektivische Ansicht einer weiteren Ausführung der Einstechvorrichtung mit einem Support mit Lagerzapfen;
- Fig. 12: zeigt eine Schnittansicht der Ausführung aus Figur 11;
- Fig. 13: zeigt eine perspektivische Ansicht einer weiteren Ausführung der Einstechvorrichtung mit einem Sicherungselement mit einem Loch für den Lagerzapfen;
- Fig. 14: zeigt eine perspektivische Ansicht des Sicherungselements der Ausführung aus Figur 13;
- Fig. 15: zeigt eine Schnittansicht der Ausführung aus Figur 13;
- Fig. 16: zeigt eine perspektivische Ansicht einer weiteren Ausführung der Einstechvorrichtung mit einem Support mit Stegen für die Halterung des Sicherungselements;
- Fig. 17: zeigt eine Schnittansicht der Ausführung aus Figur 16;
- Fig. 18: zeigt eine perspektivische Ansicht einer weiteren Ausführung der Einstechvorrichtung, bei der das Sicherungselement über der Leiterplatte angeordnet ist.

### FIGURENBESCHREIBUNG

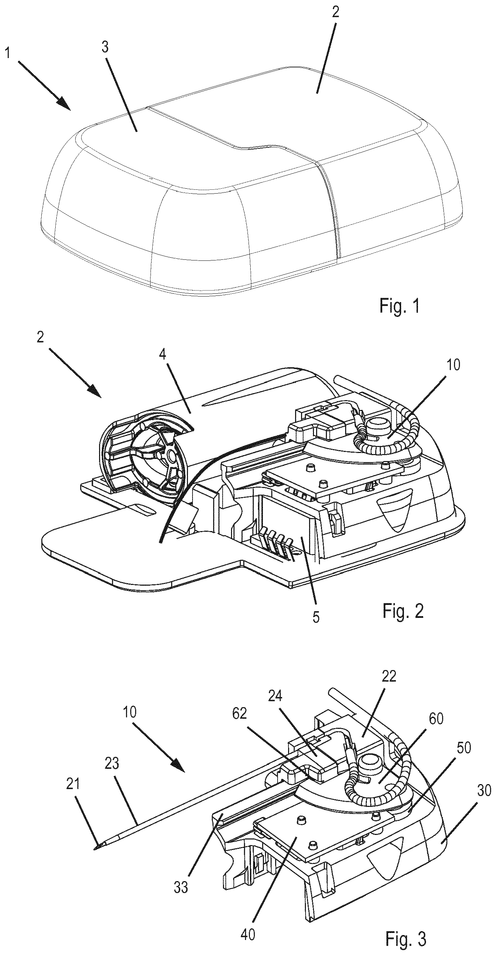

Die Figur 1 zeigt eine perspektivische Ansicht einer erfindungsgemässen Verabreichungsvorrichtung in der Form einer Patch-Pumpe 1, welche eine erfindungsgemässe Einstechvorrichtung 10 umfasst. Die Patch-Pumpe umfasst eine Einwegeinheit, nachfolgend Disposable 2 genannt, und eine Mehrwegeinheit, nachfolgen Reusable 3 genannt.

In Figur 2 ist das Disposable 2 perspektivische ohne Gehäuse der Patch-Pumpe 1 dargestellt. Das Disposable 2 beinhaltet ein mit einer medizinischen Substanz befüllbares Reservoir 4, eine Stromversorgungseinheit 5 mit einer Einwegbatterie in Form einer Knopfzelle, eine Klebefläche zur Befestigung der Patch-Pumpe 1 auf der Haut des Benutzers und eine erfindungsgemässen Einstechvorrichtung 10.

Das Reusable 3 ist über einen Bajonettverschluss lösbar und dichtend mit dem Disposable 2 kuppelbar und umfasst eine Antriebseinheit zum Antreiben einer Kolbenstange, einen Encoder, einen aufladbaren Akku sowie eine Steuerungselektronik. Der Akku kann, wenn Disposable 2 und Reusable 3 elektrisch miteinander gekoppelt sind, von der Einwegbatterie im Disposable 2 aufgeladen werden. Der Akku dient zur Stromversorgung des im Reusable 3 enthaltenen Motors sowie der Steuerungselektronik. Diese dient zur Ansteuerung des Motors und kann Daten mit einem externen Steuergerät z. B. über eine Funkverbindung austauschen. Der Motor kann über die Kolbenstange mechanisch auf das Reservoir im Disposable 2 einwirken, um die darin enthaltene medizinische Substanz auszuschütten. Weiter liefert der Akku die elektrische Energie für die Auslösung des Einstechvorgangs wie weiter unten beschrieben.

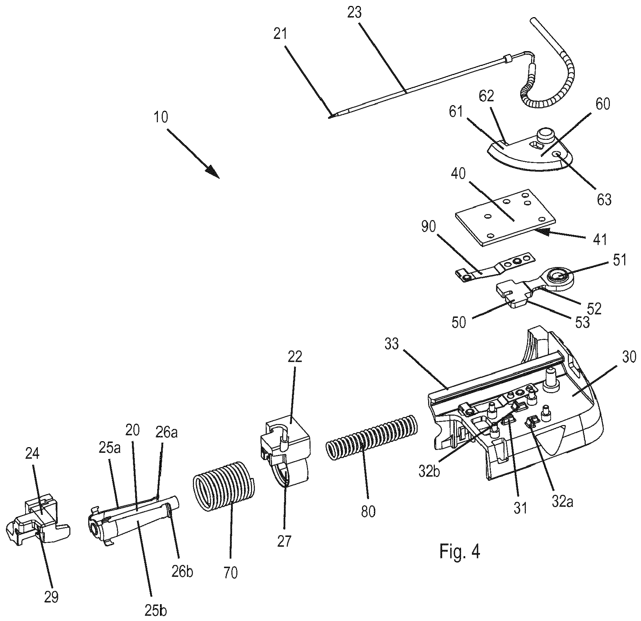

Nachfolgend werden die Bestandteile der Einstechvorrichtung 10 anhand der Figuren 3 und 4 erläutert. Figur 3 zeigt eine perspektivische Gesamtansicht der erfindungsgemässen Einstechvorrichtung 10. In dieser Darstellung sind eine Hart- und Softkanüle 21, 23 ersichtlich, welche jedoch in einem Initialzustand vor der Montage gezeigt sind, in dem die Kanülen nicht gebogen sind. In Figur 4 ist eine Explosionsansicht der Einstechvorrichtung 10 darstellt.

Die Einstechvorrichtung 10 umfasst eine Kanüleneinheit beinhaltend einen Führungszylinder 20 mit zwei seitlichen Armen 25a, 25b, die Hartkanüle 21, die Softkanüle 23 sowie einen Hartkanülenhalter 22 und einen Softkanülenhalter 24, welche die Hart- resp. Softkanüle 21, 23 halten und relativ zu einem Support 30 verschieben können. Die Arme 25a, 25b des Führungszylinders 20 sind an einem proximalen Ende des Führungszylinders 20 mit diesem verbunden. Die Arme 25a, 25b verlaufen dabei entlang einer Längsachse des Führungszylinders 20 und sind in einem distalen Bereich in radialer Richtung gegenüber dem Führungszylinder 20 bewegbar. Am distalen Ende der Arme 25a, 25b weisen diese je eine radial nach aussen weisende Lasche 26a, 26b auf, erkennbar in Figur 4. Die Einstechvorrichtung 10 umfasst zudem ein elastisches Element als Einstechfeder 80 in der Form einer Druckfeder realisiert, mit der die Kanüleneinheit in eine Einstechrichtung vorgespannt ist, ein zweites elastisches Element als Rückzugsfeder 70 ebenfalls in der Form einer Druckfeder realisiert, den Support 30, eine auf dem Support 30 befestigte Leiterplatte 40, ein zwischen dem Support 30 und der Leiterplatte 40 angeordnetes Sicherungselement 50 und ein Rückhalteelement 60, welches mit dem Sicherungselement 50 verbunden ist, die Vorspannkraft auf das Sicherungselement 50 übertragen kann sowie die vorgespannte Kanüleneinheit in einer Sicherungsstellung halten kann. Auf dem Support 30 ist ferner ein Positionsdetektor 90 in der Form eines geformten Blechstreifens angeordnet.

Die Einstechvorrichtung 10 ist in der gezeigten Ausführungsform als Kanülen-Einstech- und Rückzugsmechanismus ausgebildet und kann nach einer Auslösung mittels der Einstechfeder 80 automatisch die Hartkanüle 21 zusammen mit der die Hartkanüle 21 umgebenden Softkanüle 23 ausschieben und nach erfolgtem Ausschubvorgang mittels der Rückzugfeder 70 die Hartkanüle 21 innerhalb der Softkanüle 23 zurückziehen. Dabei verbleibt die ausgefahrene Softkanüle 23 im ausgefahrenen Zustand. Werden Hart- und Softkanüle 21, 23 vor dem Ausschieben auf einen Körper des Benutzers aufgebracht, kann so ein automatischer Einstechvorgang realisiert werden.

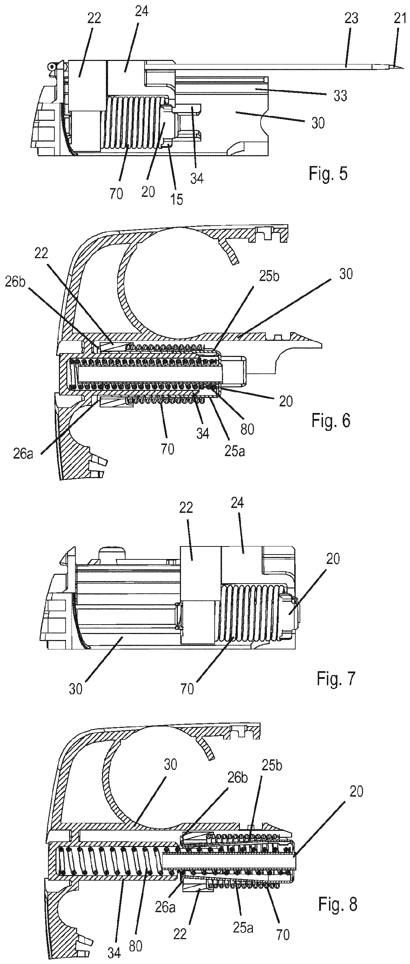

Die Figuren 5 und 6 zeigen die Einstechvorrichtung 10 in einer Sicherungsstellung der Einstecheinheit. Figur 5 ist eine Seitenansicht der Einstechvorrichtung 10, Figur 6 ist eine Schnittansicht der Einstechvorrichtung 10, wobei die Schnittebene horizontal durch die Einstechvorrichtung 10 und durch die Mittelachse der Einstechfeder 80 und der Rückzugsfeder 70 verläuft.

Die Softkanüle 23 ist koaxial und dichtend über der Hartkanüle 21 angeordnet. In der Sicherungsstellung der Kanüleneinheit ragt eine geschliffene Spitze der Hartkanüle 21 um wenige Millimeter aus der Softkanüle 23 heraus, um ein möglichst reibungsloses und schmerzfreies Einstechen in die Haut zu ermöglichen. Die Softkanüle 23 liegt mit ihrem Innenumfang dichtend am Aussenumfang der Hartkanüle 21 an, sodass keine Flüssigkeit durch den Bereich hindurchtreten kann, an welchem sich Hartkanüle 21 und Softkanüle 23 berühren. Dabei ist die Hartkanüle 21 innerhalb der Softkanüle 23 verschiebbar. Die Softkanüle 23 ist aus einem flexiblen Kunststoff wie beispielsweise einem Fluorpolymer wie FEP gefertigt, während die Hartkanüle 21 steifer ist und vorzugsweise aus Stahl besteht. Die Soft- und Hartkanüle 21, 23 sind je biegbar und sind in ihrer Längsrichtung gebogen oder gekrümmt.

Die Softkanüle 23 ist am Softkanülenhalter 24 mittels einer formschlüssigen Verbindung unbewegbar gehalten. Hierzu weist der Softkanülenhalter 24 einen nach oben offenen Kanal auf, in dem die Hart- und Softkanüle 21, 23 eingelegt sind. Am oberen Abschluss weisen die Kanalwände zur Kanalmitte weisende Auskragungen auf, wodurch sich die Hart- und Softkanüle 21, 23 nicht nach oben aus dem Kanal wegbewegen können. Die Softkanüle 23 weist zudem über einen Abschnitt eine Verdickung auf, welche in einen entsprechend geformten Kanalabschnitt passt. Dadurch ist die Softkanüle 21 in Längsrichtung formschlüssig im Kanal des Softkanülenhalters gehalten. Der Softkanülenhalter 24 ist zusammen mit der Softkanüle 23 relativ zum Support 30 an einer Führung 33 des Supports 30 entlang einer Längsrichtung des Supports 30 verschiebbar. Die Führung 33 ist im Querschnitt T-förmig ausgebildet und der Softkanülenhalter 24 weist eine entsprechend T-förmige Öffnung auf, mit welcher er an der Führung 33 gehalten und geführt ist.

Wie in Figur 3 ersichtlich, befindet sich in der Sicherungsstellung der Kanüleneinheit am distalen (nicht am einstechseitigen) Abschluss des Softkanülenhalters 24 anschliessend der Hartkanülenhalter 22, welcher die Hartkanüle 21 ebenfalls über eine formschlüssige Verbindung unbewegbar hält. Dazu weist der Hartkanülenhalter 22 einen nach oben offenen bogenförmigen Kanal auf, in dem die Hartkanüle 21 eingelegt ist. Wie beim Softkanülenhalter 24 hindern Auskragungen im Kanal des Hartkanülenhalters 22 die Hartkanüle 21 an einer Bewegung nach oben und halten dadurch die Hartkanüle 21 im Kanal. Aufgrund der Bogenform des Kanals ist die Hartkanüle 21 auch in Längsrichtung formschlüssig im Hartkanülenhalter 22 gehalten. Der Hartkanülenhalter 22 ist über eine T-förmige Öffnung auf der Führung 33 des Supports 30 verschiebbar geführt. Zudem umgibt der Hartkanülenhalter 22 mittels einer kreisrunden Öffnung 27 einen horizontalen, zylinderförmigen Dorn 34 des Supports 30. Der Dorn 34 ist an einem Ende mit dem Support 30 verbunden und ist am anderen Ende frei. Dieser horizontale Dorn 34 lagert den Führungszylinder 20 in Längsrichtung verschiebbar. Der Dorn 34 weist eine horizontale Bohrung auf, in der sich die Einstechfeder 80 befindet. Diese stützt sich am distalen Ende am Grund der Bohrung ab und am einstechseitigen Ende an einer proximalen Abschlusswand des Führungszylinders 20, ersichtlich in Figur 6.

Um den Führungszylinder 20, dessen Umfangswandung den Dorn 34 umschliesst, ist die Rückzugsfeder 70 angeordnet. Wie in Figur 6 ersichtlich, schlägt die Rückzugsfeder 70 mit ihrem distalen Ende am Hartkanülenhalter 22 an und stützt sich mit ihrem proximalen oder einstechseitigen Ende an radialen Auskragungen 15 des Führungszylinders 20 ab. Die Rückzugsfeder 70 ist somit koaxial und in Längsrichtung gesehen überlappend zur Einstechfeder 80 platziert. Die Rückzugsfeder 70 ist in der Sicherungsstellung komprimiert.

Auch die Einstechfeder 80 ist in der Sicherungsstellung komprimiert, erkennbar in Figur 6 und erzeugt dadurch eine Vorspannkraft, welche auf den Führungszylinder 20 wirkt. Der Führungszylinder 20 übertragt die Vorspannkraft mittels seiner Arme 25a, 25b und weiter über die Laschen 26a, 26b der Arme auf den Hartkanülenhalter 22. Dieser liegt mit einer proximalen Fläche am Softkanülenhalter 24 an, wodurch die Vorspannkraft auf diesen übertragen wird. Der Softkanülenhalter 24 wiederum stützt sich an einer Nase 61 des Rückhalteelementes 60 ab.

Somit wird die gesamte vorgespannte Kanüleneinheit durch das Rückhalteelement 60 an einer Bewegung in Einstechrichtung gehindert. Hierzu weist der Softkanülenhalter 24 eine Kontaktfläche 29 auf, erkennbar in Figur 4, welche schräg zur Längsachse der Kanülen oder schräg zur Bewegungsrichtung des Softkanülenhalters 24 ausgerichtet ist. Die Kontaktfläche 29 schlägt in der Sicherungsstellung an einer Sicherungsfläche 62 der auskragenden Nase 61 des Rückhalteelements 60 an. Das Rückhalteelement 60 hat im Wesentlichen die Form eines Kreissektors von etwas weniger als 90° und ist drehbar am Support 30 und an einem Gehäuse der Patch-Pumpe 1 gelagert. Es lässt sich ungefähr um 12° relativ zum Support 30 verschwenken. Dabei sind die Nase 61 auf einer Seite des Kreissektors und ein Zapfen 63 auf einer gegenüberliegenden Seite des Kreissektors angeordnet. Wie nachfolgend ausgeführt, wirkt das Rückhalteelement 60 als Hebel.

In der Sicherungsstellung der Kanüleneinheit hält das Rückhalteelement 60 mit seiner Nase 61 über die Sicherungsfläche 62 die vorgespannte Kanüleneinheit in einer distalen Position. Die Vorspannkraft, welche über die Kanüleneinheit auf das Rückhalteelement 60 einwirkt, überträgt das Rückhalteelement 60 zumindest teilweise auf das Sicherungselement 50, welches das Rückhalteelement 60 in Position hält und an einer Drehbewegung hindert. Hierzu greift der Zapfen 63 des Rückhalteelements 60 in eine kreisrunde Öffnung 51 im Sicherungselement 50 ein, siehe Figuren 3 und 4. Dadurch blockiert das Sicherungselement 50 das Rückhalteelement 60 und hält somit die gesamte Kanüleneinheit in Position. Mit anderen Worten überträgt das Rückhalteelement 60 die durch die komprimierte Einstechfeder 80 erzeugte Vorspannkraft teilweise auf das Sicherungselement 50 und teilweise auf den Support 30.

Das Sicherungselement 50 hat eine längliche Form und ist aus amorphem Kunststoff gefertigt. An einem einstechseitigen oder proximalen Ende weist das Sicherungselement 50 eine horizontal durchgehende Nut auf. In diese Nut greift ein Nocken 31 des Supports ein und stützt das Sicherungselement 50 dadurch in proximaler Richtung ab. Zudem wird das Sicherungselement 50 durch die Nut und den Nocken 31 gegen Verdrehung gesichert. Wie weiter unten im Detail erläutert, weist das Sicherungselement 50 seitlich je eine Schrägfläche 53 auf, welche auf Gegenflächen 32a, 32b des Supports 30 aufliegen und dadurch das Sicherungselement 50 an einer Bewegung in distaler Richtung hindern.

Am distalen Ende des Sicherungselements 50 befindet sich die kreisrunde Öffnung 51, welche den Zapfen 63 des Rückhalteelements 60 aufnimmt. In der Mitte zwischen den beiden Enden weist das Sicherungselement 50 eine Verjüngung 52 auf, welche gegenüber den anderen Bereichen des Sicherungselements einer kleineren Querschnittsfläche aufweist. Dieser Bereich mit der Verjüngung 52 wird bei einer Auslösung (siehe unten) erwärmt. Er kann daher auch als thermosensitiver Bereich bezeichnet werden.

Auf Sockeln des Supports 30 und über dem Sicherungselement 50 ist eine Leiterplatte 40 (PCB) durch Heissverstemmen am Support 30 befestigt. Die Leiterplatte 40 weist ein flächenförmiges Heizelement 41 auf, das in die Leiterplatte 40 integriert ist. Das Heizelement 41 ist als elektrischer Widerstand realisiert und aus einer Polymerpaste gebildet. Das Heizelement 41 weist Fläche von ungefähr 2 mm² auf und hat eine Dicke von ungefähr 20 µm. Der elektrische Widerstand beträgt je nach Ausführung zwischen 10 und 30 Ohm, wobei eine Wärmeleistung von ungefähr 300 bis 650mW erzielt wird.

Wie in Figur 3 ersichtlich, ist das Sicherungselement 50 zwischen einer Oberfläche des Supports 30 und einer Unterseite der Leiterplatte 40 platziert. Leiterplatte 40 und Sicherungselement 50 sind dabei derart angeordnet, dass sich der Bereich mit der Verjüngung 52 direkt unter dem Heizelement 41 der Leiterplatte 40 befindet.

Die Schrägflächen 53 des Sicherungselements 50 stehen in einem Winkel von ungefähr 20 zur Lotrichtung, erkennbar in Figur 4. Die Gegenflächen 32a, 32b des Supports 30 weisen den gleichen Winkel auf. In der Sicherungsstellung wird durch die übertragene Vorspannkraft das Sicherungselement 50 mit seinen Schrägflächen 53 gegen die Gegenflächen 32a, 32b des Supports 30 gezogen, so dass die Schrägflächen 53 des Sicherungselements 50 auf den Gegenflächen 32a, 32b nach oben vom Support 30 weg leicht abrutschen oder abgleiten. Dadurch wird das Sicherungselement 50 gegen die Leiterplatte 40 gedrückt und gleichzeitig gegen eine distale Bewegung gehalten. Die Verjüngung 52 des Sicherungselements 50 wird hierbei auf das Heizelement 41 in der Leiterplatte 40 gedrückt. Ein Luftspalt zwischen Verjüngung 52 und Heizelement 41 wird somit wirksam vermieden.

In der Sicherungsstellung liegt der Positionsdetektor 90 mit einem Bereich auf der Leiterplatte 40 auf, wodurch ein Kontakt zwischen dem Blech des Positionsdetektors 90 und einer Leiterplattenkontaktfläche hergestellt wird. Die Steuerung der Patch-Pumpe 1 erhält ein Signal, dass sich die Kanüleneinheit in der Sicherungsstellung befindet.

Um den Einstechvorgang auszulösen, so dass die Hart- und Softkanüle 21, 23 zum Einstechen in Einstechrichtung bewegt werden, wird über die Steuerung der Patch-Pumpe 1 das Heizelement 41 mit Strom versorgt. Durch den elektrischen Widerstand erwärmt sich das Heizelement 41. Infolge dieser Erwärmung wird die Verjüngung 52 des Sicherungselements 50 auf ca. 100°C erwärmt und der amorphe Kunststoff des Sicherungselements beginnt sich zu verformen. Durch die von der Einstechfeder 80 erzeugte Vorspannkraft wird die erhitzte Verjüngung 52 gedehnt oder das Sicherungselement 50 reisst an der Stelle der Verjüngung 52 auseinander. Dadurch kann das Rückhalteelement 60 nicht mehr durch das Sicherungselement 50 blockiert werden, die vorgespannte Einstechfeder 80 kann sich infolge entspannen und bewegt die Kanüleneinheit in Einstechrichtung. Bei dieser Bewegung drückt oder drängt die Kanüleneinheit über ihre schräg zur Bewegungsrichtung angeordnete Kontaktfläche 29 die Nase 61 des Rückhalteelements 60 weg, so dass sich das Rückhalteelement 60 um seine Drehachse verschwenkt. Die Einstechfeder 80 kann sich weiter entspannen und verschiebt dabei die Kanüleneinheit inklusive der Hart- und Softkanüle 21, 23, der Hart- und Softkanülenhalter 22, 24, des Führungszylinders 20 und der vorgespannten Rückzugsfeder 70 in Einstechrichtung. Die Hart- und Softkanüle 21, 23 werden also gleichzeitig zur Injektionsstelle hin bewegt.

Die Kanüleneinheit wird dabei bis zu einem Anschlag bewegt, bei dem sie sich in einer Einstechstellung befinden. Die Figuren 7 und 8 zeigen die Kanüleneinheit in dieser Einstechstellung, wobei Figur 7 eine Seitenansicht zeigt, bei der die Hart- und Softkanüle 21, 23 nicht dargestellt sind. Figur 8 ist eine Schnittansicht, bei der die Schnittfläche horizontal und durch die Mittelachsen der Einstechfeder 80 und der Rückzugsfeder 70 durch die Einstechvorrichtung 10 verläuft.

In der Einstechstellung wird ein einstechseitiges Ende des Blechs des Positionsdetektors 90 durch eine Anschrägung am Softkanülenhalter abgelenkt, so dass das Blech des Positionsdetektors 80 keinen Kontakt mehr hat zur Kontaktfläche auf der Leiterplatte. Dadurch wird ein Kontaktschalter geöffnet. Die Steuerung der Patch-Pumpe 1 kann dies als Signal interpretieren, dass sich die Kanüleneinheit in der Einstechstellung befindet.

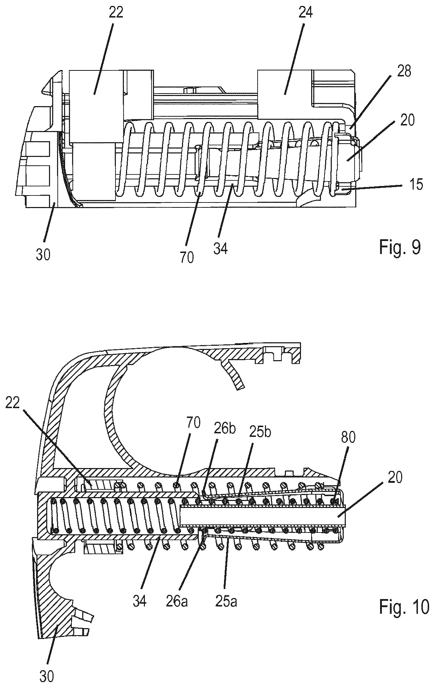

Während der Verschiebung in Einstechrichtung werden die bewegbaren seitlichen Arme 25a, 25b des Führungszylinders 20 auf dem Dorn 34 des Supports 30 entlang geführt. Der Dorn 34 ist jedoch im Bereich, in dem die Arme 25a, 25b auf dem Dorn 34 aufliegen, etwas kürzer als der Verschiebeweg der Kanüleneinheit, so dass vor dem Anschlag der Kanüleneinheit die radial nach innen vorgespannten Arme 25a, 25b des Führungszylinders 20 am Ende des Bereichs des Dorns 34 abgleiten und sich radial nach innen bewegen können. Das Ableiten der Arme 25a, 25b wird durch die Federkräfte der Einstechfeder 80 wie auch der Rückzugsfeder 70 unterstützt. Durch das Abgleiten werden auch die Laschen 26a, 26b der Arme 25a, 25b radial nach innen bewegt (Figur 8 und 10), wodurch der Hartkanülenhalter 22 vom Führungszylinder 20 freigegeben wird. Die Rückzugsfeder 70 kann sich infolge entspannen und verschiebt den Hartkanülenhalter 22 in distaler Richtung zurück in die distale Ausgangsstellung, wodurch die mit dem Hartkanülenhalter 22 verbundene Hartkanüle 21 innerhalb der Softkanüle 23 zurückgezogen wird, so dass nur die Softkanüle 23 im ausgefahrenen Zustand verbleibt. Diese Rückzugstellung der Kanüleneinheit ist in den Figuren 9 und 10 dargestellt. Figur 9 zeigt eine Seitenansicht, bei der die Hart- und Softkanüle 21, 23 nicht dargestellt sind, während Figur 9 eine Schnittansicht mit einer horizontal durch die Einstechvorrichtung 10 verlaufenden Schnittebene zeigt.

Wie in Figur 9 und 10 erkennbar, hat sich die Rückzugsfeder 70 entspannt und hat dadurch den Hartkanülenhalter 22 zusammen mit der Hartkanüle 21 wieder in die initiale, distale Position zurückverschoben. In dieser Rückzugstellung stützt sich die Rückzugsfeder 70 am distalen Ende am zurückgezogenen Hartkanülenhalter 22 und am proximalen Ende an den Auskragungen 15 des Führungszylinders 20 ab. Am proximalen Ende der Rückzugsfeder 70 liegt der Softkanülenhalter 24 mit seinem Anschlag 28 an der Rückzugsfeder 70 an. Dadurch hält die Rückzugsfeder 70 den Softkanülenhalter 24 mit der Softkanüle 23 in der proximalen Einstechstellung. Durch die entspannte Einstechfeder 80 wird der Führungszylinder 20 in der proximalen Einstechstellung gehalten. In einer alternativen Ausführung besteht auch die Möglichkeit, dass die entspannte Einstechfeder 80 den Softkanülenhalter 24 mit der Softkanüle 23 in der Einstechstellung hält.

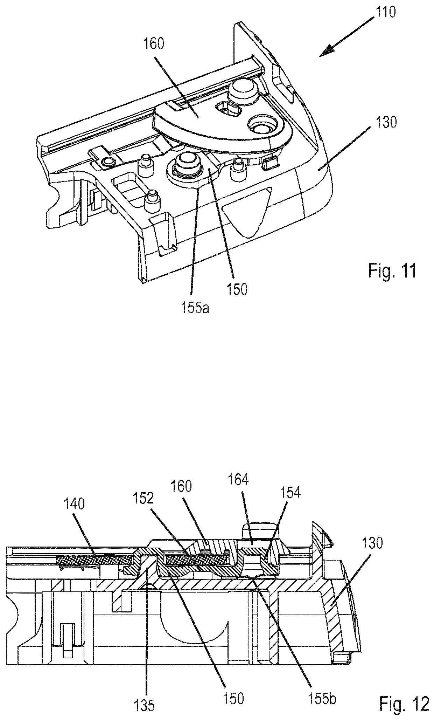

Die Figur 11 zeigt eine perspektivische Ansicht einer weiteren Ausführung der erfindungsgemässen Einstechvorrichtung 110. Dabei ist in der Figur 11 der Support 130 mit dem Sicherungselement 150 und dem Rückhalteelement 160 ersichtlich. Hingegen sind die Leiterplatte, die Kanülen und die Kanülenhalter der Übersichtlichkeit halber nicht dargestellt.

Im Unterschied zur oben beschriebenen Ausführung ist das Sicherungselement 150 in anderer Weise auf dem Support 130 gelagert und gehalten. Zudem weisen, wie nachfolgend im Detail beschrieben, Sicherungselement 150 und Rückhalteelement 160 eine andere Form auf als in der Ausführung gemäss Figuren 2 - 10.

In der Figur 12 ist eine Schnittansicht der Ausführung gemäss Figur 11 dargestellt, wobei die Schnittebene vertikal durch die Leiterplatte 140, das Sicherungselement 150, den Support 130 und durch die Mittelachse eines Lagerzapfens 135 des Supports 130 verläuft. In Figur 12 ist erkennbar, dass das Sicherungselement 150 einerseits auf dem konischen Lagerzapfen 135 des Supports 130 aufliegt und somit an dieser Stelle am Support 130 gehalten ist und andererseits mit einem gegenüber dem Support 130 frei bewegbaren Ende in eine Öffnung 164 im Rückhalteelement 160 eingreift. Hierzu weist das Sicherungselement 150 einen kegelstumpfförmigen Zapfen 154 auf, welcher in die kreisrunde Öffnung 164 im Sicherungselement 150 eingeführt ist. Auf der Unterseite weist das Sicherungselement in seinem proximalen und distalen Bereich je ein quer über die Unterseite des Sicherungselements 150 verlaufenden Steg 155a, 155b auf. Der proximale Steg 155a ist in Figur 11, der distale Steg 155b in Figur 12 ersichtlich. Mit diesen Stegen 155a, 155b liegt das Sicherungselement 150 auf einer Oberfläche des Supports 130 auf.

Die Leiterplatte 140, die sich über dem Sicherungselement 150 befindet, weist eine kreisrunde Aussparung oder Loch auf, durch welches der Lagerzapfen 135 und der darauf aufliegende Bereich des Sicherungselements 150 hindurch ragen, erkennbar in Figur 12.

Durch die zumindest teilweise übertragene Vorspannkraft von der Einstechfeder 80 auf das Sicherungselement 150 und die geometrische Form des Sicherungselements 150, wird dieses gezielt verformt. Die Krafteinleitung vom Rückhalteelement 160 auf das Sicherungselement 150 erfolgt in senkrechter Richtung gesehen oberhalb der neutralen Faser im Längsquerschnitt der Verjüngung 152 des Sicherungselements 150. Dadurch entsteht ein Drehmoment, welches ein nach oben Biegen des Sicherungselements 150 bewirkt. Die Verjüngung 152 des Sicherungselements 150 wird dadurch nach oben an eine Unterseite der Leiterplatte 140 gedrückt. Wenn sich das Heizelement erwärmt, wird das Sicherungselement 150 im Bereich seiner Verjüngung 152 geschwächt oder aufgeschmolzen, so dass das vorgespannte Rückhalteelement 160 sich drehen kann und dadurch, wie oben beschrieben, der Einstechvorgang auslöst wird. Da das Sicherungselement 150 drehbar am Support 130 gelagert ist, kann es sich bei der Bewegung des Rückhalteelements 160 ebenfalls leicht drehen, wodurch die Verjüngung 152 gleichmässig auf das Heizelement gedrückt wird. Dies ermöglicht ein optimaler Wärmeübergang vom Heizelement auf das Sicherungselement 150.

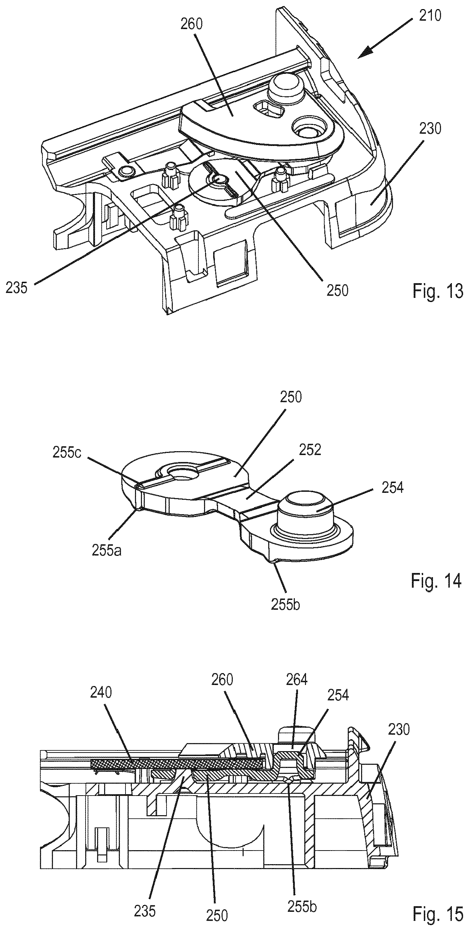

Eine weitere Ausführung der erfindungsgemässen Einstechvorrichtung 210 zeigen die Figuren 13 bis 15. Dabei sind in Figur 13 die Leiterplatte, die Kanülen und die Kanülenhalter der Übersichtlichkeit halber nicht dargestellt. Im Unterschied zur in den Figuren 11 und 12 dargestellten Ausführung, ist der Lagerzapfen 235 in der Ausführung gemäss Figur 13 bis 15 kleiner ausgebildet und das Sicherungselement 250 weist ein Loch auf, welches den Lagerzapfen 235 umschliesst. Dadurch wird das Sicherungselement 250 am Support 230 gehalten. Somit ist keine Öffnung in der Leiterplatte 240 vonnöten, was deren Herstellung vereinfacht.

In Figur 14 ist das Sicherungselement 250 dieser Ausführung in einer perspektivischen Ansicht einzeln und etwas grösser dargestellt. Wie in Figur 14 erkennbar, weist das Sicherungselement 250 auf seiner Unterseite im proximalen und distalen Bereich je einen im Querschnitt halbrunden Steg 255a, 255b auf, mit denen das Sicherungselement 250 auf der Oberfläche des Supports 230 aufliegt. Gegenüber der über dem Sicherungselement 250 angeordneten Leiterplatte 240 stützt sich das Sicherungselement mit einem auf seiner Oberfläche befindlichen Steg 225c ab. Zudem ist in Figur 14 erkennbar, dass im Bereich der Verjüngung 252, welche sich zwischen dem Loch und dem Zapfen 254 befindet, sowohl die Breite wie auch die Dicke des Sicherungselements 250 reduziert ist.

In der Figur 15 ist diese Ausführung der Einstechvorrichtung 210 in einer Schnittansicht dargestellt. Wie ersichtlich, ist das Sicherungselement 250 mit einem Ende am Lagerzapfen 235 und mit dem anderen, gegenüber dem Support 230 frei beweglichen Ende in der Öffnung 264 im Rückhalteelement 260 gehalten.

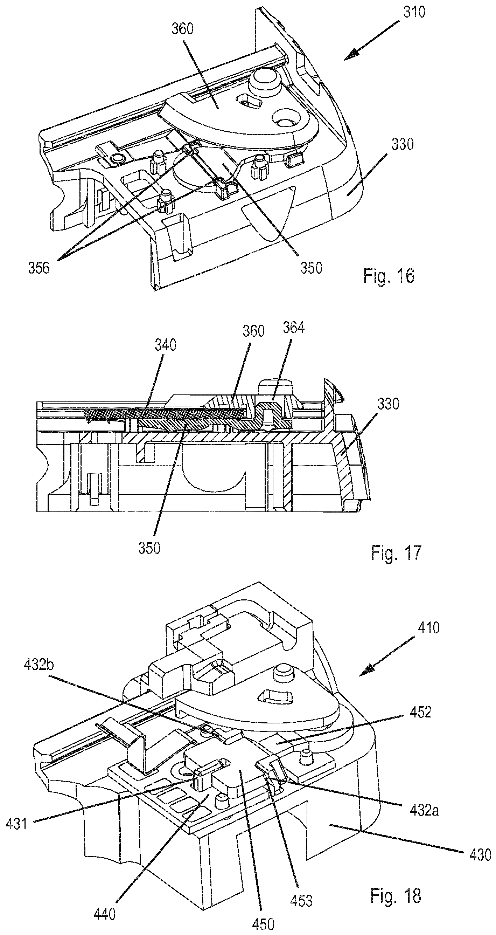

Die Figuren 16 und 17 zeigen wieder eine andere Ausführung der erfindungsgemässen Einstechvorrichtung 310, bei der das Sicherungselement 350 an einem Ende auf beiden Seiten je eine vertikale Nut 356 umfasst. Je ein Steg des Supports 330 ragt in eine Nut 356. Dadurch wird das Sicherungselement 350 relativ zum Support 330 in Richtungen parallel zur Oberfläche des Supports 330 unbeweglich gehalten. Mit seinem anderen, gegenüber dem Support 330 freien Ende ist das Sicherungselement 350 in der Öffnung 364 des Rückhalteelements 360 gehalten, wie bei den vorherigen Ausführungen gemäss Figur 11 bis 15 beschrieben. Bei der Ausführung gemäss Figur 16 und 17 ist wie bei der Ausführung gemäss Figuren 14 und 15 keine Aussparung oder Loch in der Leiterplatte 340 nötig. Durch die die beiden Nuten 356 kann sich das Sicherungselement 350 nicht relativ zum Support 330 verdrehen.

Bei einer weiteren erfindungsgemässen Ausführung der Einstechvorrichtung 410, dargestellt in Figur 18, ist im Unterschied zu den in den Figuren 1 bis 17 gezeigten Ausführungen das Sicherungselement 450 über der Leiterplatte 440 platziert. Somit ist die Leiterplatte 440 zwischen Support 430 und Sicherungselement 450 angeordnet. Dabei weist das Sicherungselement 450 Schrägflächen 453 auf, welche mit Gegenflächen 432a, 432b des Supports 430 zusammenwirken. Das Sicherungselement 450 wird in dieser Ausführung durch die Vorspannung nach unten auf die Leiterplatte 440 gedrückt, so dass die Verjüngung 452 des Sicherungselements 450 von oben auf das Heizelement gedrückt wird. Die Lagerung des Sicherungselement 450 auf dem Support 430 erfolgt dabei über eine Nut im Sicherungselement 450 und einen Nocken 431 des Supports 430 wie bei der Ausführung gemäss Figur 1 - 10 beschrieben.

### BEZUGSZEICHENLISTE

- 1: Patch-Pumpe
- 2: Disposable
- 3: Reusable
- 4: Reservoir
- 5: Stromversorgungseinheit
- 10, 110, 210, 310, 410: Einstechvorrichtung
- 15: Auskragungen
- 20: Führungszylinder
- 21: Hartkanüle
- 22: Hartkanülenhalter
- 23: Softkanüle
- 24: Softkanülenhalter
- 25a, 25b: Arme
- 26a, 26b: Laschen
- 27: Öffnung
- 28: Anschlag
- 29: Kontaktfläche
- 30, 130, 230, 330, 430: Support
- 31, 431: Nocken
- 32a, 32b, 432a, 432b: Gegenflächen
- 33: Führung
- 34: Dorn
- 40, 140, 240, 340, 440: Leiterplatte
- 41: Heizelement
- 50, 150, 250, 350, 450: Sicherungselement
- 51: Öffnung
- 52, 152, 252, 452: Verjüngung
- 53, 453: Schrägfläche
- 60, 160, 260, 360: Rückhalteelement
- 61: Nase
- 62: Sicherungsfläche
- 63: Zapfen
- 70: Rückzugsfeder
- 80: Einstechfeder
- 90: Positionsdetektor
- 135, 235: Lagerzapfen
- 154, 254: Zapfen
- 155a, 155b,: Steg
- 255a, 255b, 255c: Steg
- 164,264,364: Öffnung
- 356: Nut

## Patentansprüche

1. Einstechvorrichtung (10) für eine Verabreichungsvorrichtung (1) umfassend
a. ein elastisches Element (80), mit dem eine Vorspannkraft zum Einstechen erzeugbar ist;
b. eine bewegliche Kanüleneinheit, welche mit der Vorspannkraft in eine Einstechrichtung vorgespannt ist oder in Einstechrichtung bewegbar ist;
c. ein Sicherungselement (50), welches die vorgespannte Kanüleneinheit in einer Sicherungsstellung halten kann und einen thermosensitiven Bereich (52) aufweist, welcher sich bei Erwärmung unter mechanischer Spannung verformt, und
d. ein Heizelement (41), mit dem der thermosensitive Bereich des Sicherungselements (50) erwärmbar ist, so dass sich der thermosensitive Bereich (52) des Sicherungselements (50) unter mechanischer, durch die Vorspannkraft hervorgerufener Spannung verformen kann und dadurch die Kanüleneinheit nicht mehr in der Sicherungsstellung gehalten werden kann und sich die Kanüleneinheit aus der Sicherungsstellung wegbewegen kann.

2. Einstechvorrichtung (10) nach Anspruch 1, **gekennzeichnet durch** ein bewegbares Rückhalteelement (60), welches funktional zwischen der Kanüleneinheit oder dem elastischen Element (80) und dem Sicherungselement (50) angeordnet ist und welches die Vorspannkraft vom elastischen Element (80) auf das Sicherungselement (50) übertragen kann.

3. Einstechvorrichtung (10) nach Anspruch 2, **dadurch gekennzeichnet, dass** das Rückhalteelement (60) als rotierbarer Hebel ausgebildet ist.

4. Einstechvorrichtung, nach Anspruch 2 oder 3, **dadurch gekennzeichnet, dass** das Rückhalteelement (60) ausschliesslich durch die Vorspannkraft bewegbar ist.

5. Einstechvorrichtung (10) nach einem der Ansprüche 1 bis 4, **dadurch gekennzeichnet, dass** das Sicherungselement (50) eine Schrägfläche (53) aufweist, welche in der Sicherungsstellung der Kanüleneinheit durch die Vorspannkraft derart mit einer Gegenfläche (32) zusammenwirkt, dass der thermosensitive Bereich des Sicherungselements (50) an das Heizelement (41) gedrückt wird.

6. Einstechvorrichtung (10) nach einem der Ansprüche 1 bis 5, **dadurch gekennzeichnet, dass** das Sicherungselement (50) gegenüber dem Heizelement (41) als separates Bauteil ausgebildet ist.

7. Einstechvorrichtung (10) nach Anspruch 6, **dadurch gekennzeichnet, dass** das Heizelement (41) in eine Leiterplatte (40) integriert ist.

8. Einstechvorrichtung (10) nach einem der Ansprüche 1 bis 7, **dadurch gekennzeichnet, dass** das Sicherungselement (50) eine längliche Form mit einer Verjüngung (52) im thermosensitiven Bereich aufweist und derart angeordnet ist, dass sich die Verjüngung (52) und das Heizelement (41) zumindest teilweise überlappen.

9. Einstechvorrichtung (10) nach einem der Ansprüche 1 bis 8, **dadurch gekennzeichnet, dass** die Kanüleneinheit eine erste und eine zweite Kanüle (21, 23) umfasst, wobei die zweite Kanüle (21) durch eine zweite Vorspannkraft von einem zweiten elastischen Element (70) entgegen der Einstechrichtung vorspannbar ist.

10. Einstechvorrichtung (10) nach Anspruch 9, **dadurch gekennzeichnet, dass** die Kanüleneinheit mit der ersten und der zweiten Kanüle (21, 23) und dem zweiten elastischen Element (70) von der Sicherungsstellung in eine Einstechstellung bewegbar ist und dass die zweite Kanüle (21) durch die zweite Vorspannkraft wieder aus der Einstechstellung wegbewegbar ist, wobei die erste Kanüle in der Einstechstellung verbleibt.

11. Einstechvorrichtung (10) nach Anspruch 9 oder 10, **dadurch gekennzeichnet, dass** das erste elastische Element (80) und das zweite elastische Element (70) je als Feder ausgebildet sind.

12. Einstechvorrichtung (10) nach Anspruch 11, **dadurch gekennzeichnet, dass** die Federn koaxial zueinander angeordnet sind.

13. Einstechvorrichtung (10) nach Anspruch 11 oder 12, **dadurch gekennzeichnet, dass** eine der zwei Federn in Längsrichtung der Feder mindestens teilweise innerhalb der anderen der zwei Federn angeordnet ist.

14. Einstechvorrichtung (10) nach einem der Ansprüche 10 bis 13, **gekennzeichnet durch** einen Positionssensor (90), mit dem bestimmbar ist, ob sich die Kanüleneinheit in der Einstechstellung befindet und welcher über einen Kanülenhalter (24) der Kanüleneinheit betätigbar ist.

15. Verabreichungsvorrichtung (1), insbesondere eine Infusionspumpe, umfassend einen Einwegteil (2) und einen Mehrwegteil (3), wobei der Einwegteil (2) ein Reservoir und eine fluidisch mit dem Reservoir verbundene Einstechvorrichtung (10) nach einem der Ansprüche 1 bis 14 umfasst.
